# DEMANDE DE BREVET EUROPEEN

(11) **EP 2 821 032 A1**
(43) Date de publication de la demande: **07.01.2015**
(21) Numéro de dépôt: 13174949.1
(22) Date de dépôt: 03.07.2013
(51) Int. Cl.: A61F 2/20

(54) **Stent pharyngé à maillage variable**

(71) Demandeur: Otorino, 1410 Waterloo (BE)
(72) Inventeur: Nawara, Gregory, 1410 Waterloo (BE)
(74) Mandataire: Decamps, Alain René François

(57) **Abrégé**

Stent pharyngé pour traiter l'apnée du sommeil apte à exercer une contrainte prévenant l'effondrement des différents tissus en contact avec le stent, les propriétés d'adhésion variant sur la périphérie du stent, l'adhésion du stent étant adaptée aux propriétés des différents tissus en contact avec le stent.

## Description

### Domaine de l'invention

L'invention se rapporte à un stent pharyngé pour traiter l'apnée du sommeil.

L'invention se rapporte aussi à dispositif de largage d'un tel stent.

### État de la technique

L'apnée du sommeil est un désordre respiratoire lié au sommeil qui affecterait entre 1 et 10% de la population adulte. En contribuant à un risque accru d'hypertension, d'arythmies cardiaques, d'attaques cérébrales et de pathologies cardiovasculaires variées, ce désordre est associé à une morbidité et une mortalité significatives. Le ronflement, qui constitue un autre désordre respiratoire lié au sommeil peut être associé ou non à l'apnée du sommeil.

L'apnée du sommeil est due à un effondrement des tissus mous des voies respiratoires supérieures durant le sommeil. Lorsqu'un individu sujet à l'apnée du sommeil est éveillé, les tissus de la langue et des parois du pharynx ont une tonicité permettant le passage de l'air pour la respiration. En revanche, pendant le sommeil, la musculature supportant ces tissus se relâche et ceux-ci s'effondrent, ce qui provoque un ronflement et dans les cas plus sévères une obstruction partielle ou totale des voies respiratoires. De plus, la dépression créée dans le pharynx au moment de l'inspiration ne fait qu'aggraver l'effondrement des tissus, ce phénomène renforçant encore les symptômes de l'apnée du sommeil.

On connait la demande de brevet EP1585468 qui dévoile des dispositifs à placer dans l'oropharynx pour traiter l'apnée du sommeil. Ces dispositifs ont pour vocation d'être installés de manière permanente au sein de l'oropharynx de manière à renforcer, raffermir ou rigidifier les tissus de cette région dans le but de préserver les voies respiratoires supérieures de l'obstruction à l'origine de l'apnée du sommeil. Ce document dévoile entre autres un stent pharyngé à placer dans l'oropharynx et aussi des dispositifs maillés de formes variées pouvant éventuellement se replier de manière à s'adapter à la forme de l'oropharynx. Aucune caractéristique spécifique ne semble cependant prévue pour assurer une bonne adhésion entre le dispositif et les différents tissus en contact avec celui-ci, les particularités de ces derniers variant pourtant fortement d'un point de vue structurel et fonctionnel. En particulier, la base de la langue est une région du pharynx exposée à des pressions importantes et possède un caractère mobile non négligeable, la langue étant par ailleurs un organe en partie musculaire capable d'appliquer des forces importantes sur un dispositif placé dans l'oropharynx. La muqueuse de la paroi pharyngée est quant à elle fine, fragile et sensible, de par son innervation sensorielle importante. Les spécificités de ces deux types de tissus en contact avec un dispositif placé dans l'oropharynx rendent les dispositifs de l'art antérieur difficilement utilisables, étant donné l'uniformité que ceux-ci présentent sur toute leur surface externe. Même si EP1585468 prévoit une grande variété de structures pour le dispositif à implanter dans l'oropharynx, aucune disposition particulière ne semble prévue pour prévoir une bonne adhésion simultanée du dispositif aux muqueuses linguale et oropharyngée, ce qui est pourtant une condition nécessaire pour assurer une bonne stabilité et une efficacité du dispositif.

Par ailleurs, en cas d'intolérance au dispositif placé dans la gorge ou en cas de mauvais positionnement de celui-ci, le dispositif doit être retiré de l'oropharynx. EP1585468 ne prévoit aucune mesure de retrait du dispositif, ce qui entraîne qu'il est dès lors impossible de le retirer sans blesser les muqueuses environnantes.

Le placement d'un stent pharyngé impose généralement que le patient soit sous anesthésie générale, ce qui entraine qu'il doit être relié à un respirateur afin de pouvoir suppléer à ses besoins en oxygène. L'oxygène lui est insufflé par une sonde d'intubation, qui doit être insérée par la bouche ou le nez dans la trachée pour assurer le maintien de la perméabilité des voies aériennes et permettre la ventilation mécanique. Dans le cas particulier d'une anesthésie générale en vue du placement d'un stent pharyngé, cette sonde d'intubation constitue un obstacle car elle est en partie présente à l'endroit où le stent pharyngé doit être largué. Les dispositifs de largage classiques utilisés pour le placement d'un stent dans le domaine de l'angioplastie, tels que celui dévoilé dans le document EP0408245, ne peuvent donc être utilisés en raison de ces contraintes spécifiques. Le placement d'un stent pharyngé est donc une manoeuvre délicate pour laquelle l'art antérieur n'apporte pas de solution adaptée.

### Résumé de l'invention

Un but de l'invention est de remédier aux inconvénients des dispositifs de l'art antérieur destinés à traiter l'apnée du sommeil ou des désordres respiratoires voisins tels que le ronflement, en fournissant un stent pharyngé stable et efficace apte à exercer une contrainte prévenant l'effondrement des différents tissus en contact avec le stent.

Un autre but de l'invention est de permettre un retrait aisé et inoffensif pour le patient du stent pharyngé selon l'invention.

Un autre but de l'invention est de permettre un placement aisé d'un stent pharyngé dans l'oropharynx.

A cette fin, l'invention fournit un stent pharyngé pour traiter l'apnée du sommeil apte à exercer une contrainte prévenant l'effondrement des différents tissus en contact avec le stent, les propriétés d'adhésion variant sur la périphérie du stent, l'adhésion du stent étant adaptée aux propriétés des différents tissus en contact avec le stent.

Par stent, on entend un dispositif tubulaire, qui est de préférence maillé et métallique, mais tout au long de ce texte, on n'exclut pas d'autres matériaux que les métaux de même que l'on n'exclut pas les dispositifs de structure continue, sans maillage donc. En général, un stent a pour fonction d'être inséré dans un conduit/passage du corps humain afin d'empêcher ou de compenser les effets d'une affection tendant à obstruer ce conduit/passage en se déployant dans ledit conduit/passage et cette définition est applicable au stent pharyngé selon l'invention.

Le stent pharyngé selon l'invention sera inséré au niveau de la cavité pharyngée, dans une région anatomique située au niveau de l'oropharynx et a pour vocation d'empêcher la bascule de la langue vers la partie postérieure du pharynx. Cette bascule est principalement à l'origine du syndrome d'apnée du sommeil. Le positionnement du stent pharyngé juste derrière la langue a pour conséquence le soutien permanent de la langue, et la disparition des apnées. Grâce au stent, la langue ne pourra plus s'affaisser vers le pharynx. La présence du stent permet également d'éviter le collapsus transversal de l'oropharynx souvent présent chez les patients souffrant d'apnées nocturnes. Ce stent agit donc comme un véritable ressort, permettant d'assurer à tout instant l'ouverture permanente des voies respiratoires, en exerçant une force radiale sur la base de la langue et les tissus de l'oropharynx.

Le stent pharyngé selon l'invention sera avantageusement auto-expansible. Grâce à sa mémoire de forme, il se déploie automatiquement dans la cavité pharyngée. L'utilisation d'un stent à ballonnet peut être envisagée également ; dans ce cas, c'est la pression qu'insuffle le ballonnet qui conditionne l'expansion du stent.

La structure tubulaire du stent peut présenter des variantes. En particulier, trois modes de réalisation avantageux peuvent être envisagés : le stent cylindrique, le stent évasé et le stent en « cornet à frites ». Le stent cylindrique à la même largeur en haut et en bas. Le stent évasé est plus large en haut qu'en bas pour s'adapter à certaines variantes de l'anatomie du patient. Le stent en « cornet à frite » est envisagé dans des circonstances anatomiques de très grosse langue, la forme du stent soutenant dans ce cas plus efficacement la bascule de la langue en position couchée sur le dos. Dans tous les cas, différentes dimensions du stent ont été envisagées de façon à ce qu'elles soient adaptées à l'anatomie du patient. Le choix de la mesure sera donc différent, en fonction du fait qu'il s'agit d'un homme, d'une femme, de leur corpulence, de particularités anatomiques, du fait qu'il s'agit d'un fumeur...

Selon un mode de réalisation avantageux, l'adhésion du stent pharyngé selon l'invention est adaptée par une variation de la contrainte locale exercée par le stent à sa périphérie sur les différents tissus, la contrainte locale exercée assurant une adhésion du stent aux différents tissus sans léser lesdits tissus.

Par contrainte locale, on entend la contrainte exercée localement sur le tissu par le fil constituant le maillage du stent. Cette contrainte locale doit être distinguée de la force radiale totale qui conditionne le pouvoir d'ouverture du stent pharyngé pour lutter contre l'effondrement des tissus de l'oropharynx et contre la bascule de la langue. En effet, dans le cas d'un stent à maillage à forme tubulaire d'un diamètre donné, bien que la force radiale totale du stent augmente lorsque le nombre de mailles augmente (et que donc la taille de celles-ci diminue) et que l'aire de la section du fil constituant ces mailles augmente, la pression exercée localement par un segment de fil augmente avec la taille de la maille car une même force radiale est exercée par une surface de fil plus faible. En diminuant localement la densité du maillage sur la périphérie du stent, on fait donc augmenter localement la pression exercée par le segment de fil, ce qui confère un pouvoir d'ancrage plus élevé à ce segment de fil. En dimensionnant de manière judicieuse les mailles du stent, on peut donc obtenir un pouvoir d'ancrage, et donc d'adhésion, du stent adapté aux propriétés des tissus en contact avec lui. Bien qu'un maillage ayant une densité qui varie sur la périphérie du stent constitue un moyen efficace pour faire varier localement la contrainte exercée sur le tissu, tout autre moyen équivalent pour faire varier localement la contrainte exercée par le stent peut être applicable. En particulier, un stent continu (non-maillé) muni sur sa périphérie de protubérances de tailles variables et espacées par des distances variables peut être réalisé, la contrainte locale étant dans ce cas liée aux paramètres de taille et d'espacement des protubérances puisqu'ils déterminent la surface sur laquelle est répartie la force radiale exercée par le stent sur les tissus.

Selon un mode de réalisation avantageux, la composante radiale de la contrainte locale exercée par le stent sur les tissus de la langue est supérieure à celle exercée sur les autres tissus du pharynx. Une contrainte supérieure au niveau de la base de la langue par rapport aux tissus de l'oropharynx permet une adhésion du stent plus adaptée aux spécificités respectives de la langue et de l'oropharynx. Comme expliqué plus haut, la base de la langue est une région du pharynx exposée à des pressions importantes et possède un caractère mobile non négligeable, la langue étant par ailleurs un organe en partie musculaire capable d'appliquer des forces importantes sur un dispositif placé dans l'oropharynx. La muqueuse de la paroi pharyngée est quant à elle fine, fragile et sensible, de par son innervation sensorielle importante. Ces éléments sont à l'origine du caractère avantageux du mode de réalisation du stent exposé ci-dessus. La contrainte locale à appliquer au niveau de la langue et celle à appliquer au niveau de l'oropharynx sont préférablement choisies de manière à maximiser l'adhésion du stent aux différents tissus sans causer de lésions à ces tissus.

Selon un mode de réalisation avantageux, le stent pharyngé selon l'invention comprend un maillage, les dimensions des mailles dudit maillage variant sur la périphérie du stent, le maillage de la partie du stent en contact avec la langue étant plus large que celui de la partie en contact avec les autres parois du pharynx.

Par maille, on entend, tout au long de ce texte, un motif de base se répétant spatialement de manière périodique et structurée pour former un maillage. Les mailles du stent peuvent avoir des structures variées. Ce maillage peut par exemple être un réseau métallique tissé, dont les fils sont réalisés en un matériau super-élastique, par exemple l'alliage Nitinol^{®}, qui est aujourd'hui le chef de file des matériaux utilisés pour les endo-prothèses telles que les stents. Cependant, vu la recherche constante dans le domaine des matériaux bio-compatibles, ainsi que l'essor progressif des nanotechnologies, tout autre matériel pourrait également, dans le futur, faire partie intégrante des stents. On pourrait également imaginer de travailler avec un stent partiellement siliconé, voire avec du matériel biodégradable, du cobalt-chrome, de l'or... Le stent selon l'invention peut également être enduit d'un adhésif biocompatible adapté du type de ceux connus dans l'art antérieur.

Selon un mode de réalisation de l'invention, le maillage du stent pharyngé consiste en un enchaînement de « virages en lacet » se répétant sur toute la périphérie du stent. Chaque maille correspond alors à un segment de fil comprenant deux virages. Dans ce mode de réalisation, les virages sont avantageusement d'angles plus grands pour la partie du stent en contact avec la langue, que les virages de la partie en en contact avec les muqueuses oropharyngées. De cette manière, les mailles sont plus serrées au niveau de l'oropharynx et plus espacées au niveau de la langue.

En faisant varier le maillage sur la périphérie du stent, on observe donc une différence entre la pression exercée localement par le fil constituant les mailles de plus grande taille et par celui constituant les mailles de plus petite taille. La pression exercée au niveau des mailles de plus grande taille est en effet plus élevée en raison de la concentration de la force radiale exercée par le stent sur une surface plus faible de fil. En augmentant la composante radiale de la force sur les mailles de plus grandes tailles, on obtient un ancrage plus profond de ces mailles dans la muqueuse en contact avec celles-ci. En raison de cet ancrage plus profond, l'adhésion des mailles de plus grande taille est donc plus élevée que celles de mailles de plus petite taille.

Selon un mode de réalisation avantageux, le fil du maillage de la partie en contact avec la langue a une section d'aire plus importante que le fil du maillage en contact avec les autres tissus du pharynx. Il a été observé qu'un tel choix optimisait les performances du stent pharyngé selon l'invention, un fil de section d'aire plus élevée au niveau de la langue étant plus adapté aux mouvements incessants et aux pressions exercées par la langue. Un fil de section d'aire plus faible pour la partie du stent en contact avec le pharynx confère à cette dernière une plus grande souplesse qui s'avère plus adaptée à la muqueuse fine et fragile de la paroi pharyngée.

Selon un mode de réalisation avantageux, le stent pharyngé selon l'invention n'entre pas en contact avec la partie mobile du voile du palais, et notamment la partie inférieure de la luette. Selon un autre mode de réalisation avantageux, le stent pharyngé selon l'invention a des dimensions qui lui permettent de rester à distance du mouvement à bascule de l'épiglotte. De manière particulièrement avantageuse, le stent épouse de manière circonférentielle l'ensemble de la cavité pharyngée en regard de la base de la langue, de manière à constamment y assurer une perméabilité au passage de l'air. Une fois le stent bien placé, il sera intégré à la paroi de manière à laisser librement le patient respirer, avaler, manger, tousser, éternuer...

Selon un mode de réalisation avantageux, des moyens additionnels pour assurer une bonne stabilité du stent sont prévus. Tout type de moyen pour accroître la stabilité et la longévité du stent dans l'oropharynx peuvent être envisagés.

Selon un mode de réalisation avantageux, les moyens pour assurer une bonne stabilité du stent comprennent une incurvation de la partie stent en contact avec la langue de manière à l'adapter à la morphologie de la langue. L'incurvation doit être adéquate pour épouser la forme de la langue et peut dépendre de la morphologie spécifique du patient destiné à recevoir le stent pharyngé selon l'invention.

Selon un mode de réalisation avantageux, les moyens pour assurer une bonne stabilité du stent comprennent des crochets fixés à la partie du stent en contact avec langue. Ces crochets sont avantageusement solidaires des mailles en contact avec la muqueuse linguale et leur dimension est choisie de manière à permettre un ancrage suffisant sur la langue sans causer de lésion à celle-ci.

Selon un mode de réalisation avantageux, le stent pharyngé selon l'invention comprend une structure filaire apte à étrangler le stent de manière à faciliter son retrait du pharynx. Cette structure filaire comprend avantageusement un fil parcourant la circonférence du stent via des anneaux solidaires des mailles. Dans un mode de réalisation avantageux, le maillage est enchaînement de « virages en lacet » et chaque maille comprend deux anneaux, l'un sur le bord supérieur du stent et l'autre sur son bord inférieur.

Selon un mode de réalisation avantageux, la structure filaire du stent pharyngé selon l'invention comprend deux fils traversant des anneaux solidaires des mailles du stent, chacun des deux fils longeant un bord dudit stent pharyngé, les deux fils étant munis de perles en leurs extrémités. Grâce à ces perles, les fils restent donc prisonniers des anneaux et ces perles peuvent également être utilisées comme prises destinées à être sollicitées au retrait du stent. Les perles sont avantageusement sphéroïdes mais toute autre forme permettant de remplir les deux fonctions exposées ci-dessus peut être choisie pour ces perles.

Selon un mode de réalisation avantageux, le stent pharyngé est auto-expansible et est monté sur un dispositif de largage comprenant une sonde d'intubation, un tube poussoir, et une gaine tubulaire externe, ledit stent pharyngé et le tube poussoir étant aptes à coulisser sur la sonde d'intubation, le tube poussoir se trouvant sur la partie proximale de la sonde d'intubation, le stent pharyngé se trouvant sur la partie distale de la sonde d'intubation, la gaine externe entourant ledit stent et ledit tube poussoir et étant apte à coulisser sur ceux-ci, le diamètre dudit stent lorsqu'il est déployé étant supérieur au diamètre de la sonde d'intubation.

Le dispositif de largage peut être utilisé avec un stent selon l'invention mais peut aussi être utilisé pour des stents pharyngés de l'art antérieur qui ne bénéficient pas des spécificités du stent pharyngé selon l'invention.

Le dispositif de largage permet de combiner en un même appareil une sonde d'intubation et un instrument de largage du stent pharyngé. Au lieu de chercher à contourner la sonde d'intubation, la sonde d'intubation est avantageusement utilisée comme support pour le stent pharyngé avant le largage dans l'oropharynx. Grâce au dispositif de largage selon l'invention, le placement du stent sous anesthésie générale ne pose plus de problème, la complexité de la manoeuvre devant être effectuée par le chirurgien étant grandement réduite par rapport aux techniques de l'art antérieur.

### Brève description des figures

Ces aspects ainsi que d'autres aspects de l'invention seront clarifiés dans la description détaillée de modes de réalisation particuliers de l'invention, référence étant faite aux dessins des figures, dans lesquelles :
La Fig.1 est une vue en perspective d'un mode de réalisation stent pharyngé selon l'invention.
La Fig.2 est une vue du côté du stent pharyngé de la Fig. 1.
La Fig.3 est une vue en plan du stent pharyngé des Fig. précédentes.
La Fig.4 est une vue en perspective du stent pharyngé selon l'invention muni de la structure filaire pour faciliter le retrait du stent.
La Fig. 5 est une vue en perspective du mode de réalisation de la Fig. 4 dans sa position rétractée.
La Fig. 6 est une vue schématique du dispositif de largage selon l'invention.
La Fig. 7 est une vue de la partie distale du dispositif de largage selon l'invention, avant le largage du stent.
La Fig. 8 est une vue de la partie distale du distale du dispositif de largage selon l'invention, lorque le stent est partiellement déployé.
La Fig. 9 est une vue de la partie distale du distale du dispositif de largage selon l'invention, au moment où le stent est largué.
La Fig. 10 est une est une vue de la partie distale du distale du dispositif de largage selon l'invention, après le largage du stent.

Les figures ne sont pas dessinées à l'échelle. Généralement, des éléments semblables sont dénotés par des références semblables dans les figures.

### Description détaillée de modes de réalisation particuliers

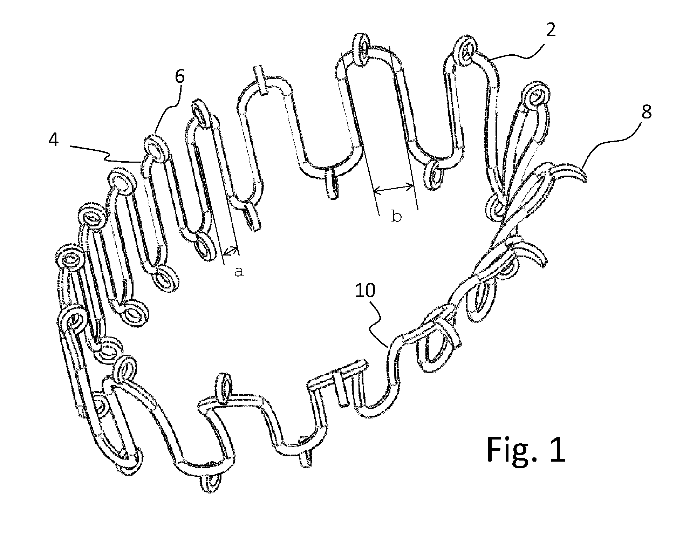

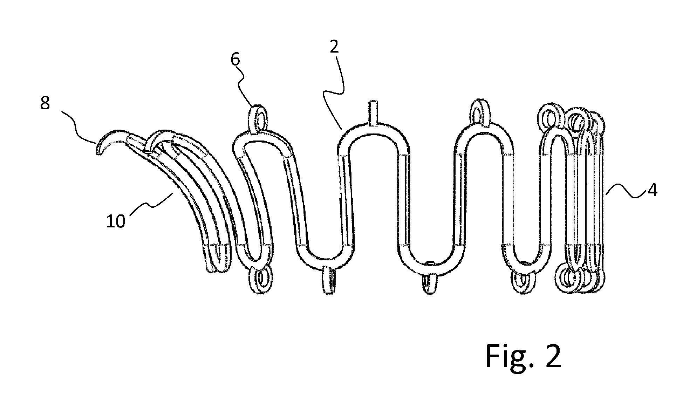

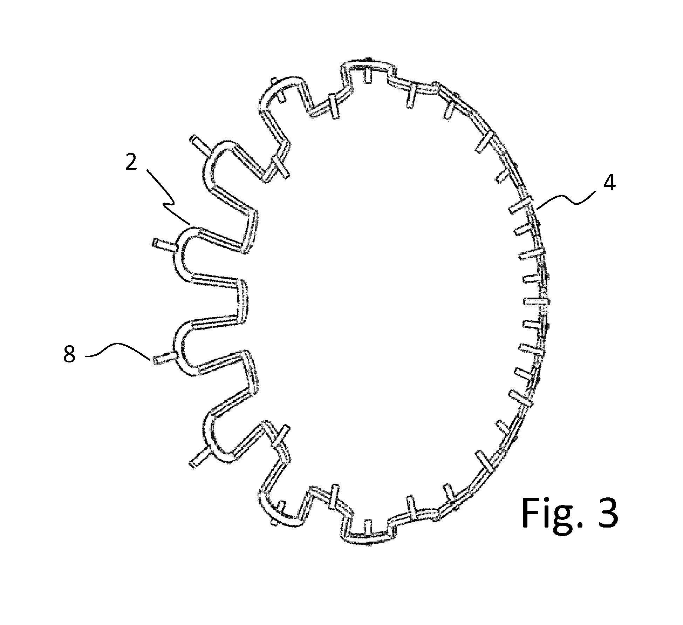

Les Fig. 1, 2 et 3 représentent un mode de réalisation du stent selon l'invention. Ce stent pharyngé est un stent auto-expansible qui doit donc être positionné dans l'oropharynx pour réduire ou même éliminer l'apnée du sommeil et/ou le ronflement, le ronflement et l'apnée du sommeil étant souvent causés par une combinaison d'une étroitesse et d'un faible tonus musculaire des voies respiratoires supérieures. La langue recule et peut obstruer les voies respiratoires, de même que d'autres régions de l'oropharynx peuvent s'effondrer.

Le stent de la Fig. 1 est donc destiné à être en partie en contact avec l'arrière de la langue et en partie avec les autres muqueuses de l'oropharynx. La partie à mailles larges 2 est la partie destinée à être en contact avec la langue et la partie à mailles plus fines 4 est la partie destinée à être en contact avec les autres muqueuses de l'oropharynx. Le maillage du stent pharyngé consiste en un enchaînement de « virages en lacet » se répétant sur toute la périphérie du stent. Les virages sont d'angles plus grands pour les mailles en contact avec la langue 2, que les virages de la partie en contact avec les muqueuses oropharyngées. De cette manière, les mailles sont plus serrées au niveau de l'oropharynx et plus espacées au niveau de la langue. Les longueurs a et b à la Fig.1 caractérisent les dimensions des deux types de maille. La valeur de a est donc inférieure à celle de b, de manière à avoir une contrainte locale exercée par le fil plus importante au niveau de la langue qu'au niveau des muqueuses oropharyngées. La longueur de chaque partie, de même que la forme du stent dans son état expansé sont étudiées de manière à se conformer au mieux à l'oropharynx du patient et à supporter ces tissus de manière à permettre une respiration substantiellement normale durant le sommeil.

Dans le mode de réalisation de la Fig. 1, l'aire de la section du fil des mailles de la partie en contact avec la langue est supérieure à celle du fil des mailles de la partie en contact avec les muqueuses oropharyngées. Un fil ayant une section d'aire plus élevée au niveau de la langue est plus adapté aux mouvements incessants et aux pressions exercées par la langue tandis qu'un fil ayant une section d'aire plus faible pour la partie du stent en contact avec le pharynx confère à cette dernière une plus grande souplesse qui s'avère plus adaptée à la muqueuse fine et fragile de la paroi pharyngée.

Le nombre total de mailles du stent, le rapport entre les longueurs a et b de mêmes que les aires des section de fil pour chacune des deux parties sont choisis de manière à optimiser la force radiale du stent tout en optimisant la contrainte locale exercée par le fil de chacune des deux parties du stent sur les tissus de la langue et de l'oropharynx. Cette contrainte locale optimale est atteinte lorsqu'une bonne adhésion du stent est obtenue sur les tissus sans leur causer de lésion.

Les mailles du stent pharyngé sont pour la plupart munie d'anneaux 6 placés à chaque virage du maillage. Ceux-ci sont destinés à permettre le passage d'une structure filaire apte à étrangler le stent de manière à faciliter son retrait en cas de nécessité. Au niveau de la partie destinée à être en contact avec la langue, les anneaux sont remplacés par des crochets d'arrimage 8 pour permettre un meilleur ancrage du stent sur la langue. La forme et les dimensions de ces crochets sont choisies de manière à permettre une fixation adéquate du stent au tissu lingual sans léser celui-ci.

Les mailles du stent pharyngé de la Fig. 1 comprennent en outre une incurvation 10 au niveau de la partie en contact avec la langue manière à s'adapter à la morphologie de la langue. L'incurvation doit être adéquate pour épouser la forme de la langue et peut dépendre de la morphologie spécifique du patient destiné à recevoir le stent pharyngé selon l'invention.

Ce stent possède par ailleurs les qualités optimales de bio-compatibilité en relation avec les tissus, alliant flexibilité, souplesse, haute conformabilité. Les propriétés physiques de sa paroi minimisent les réactions inflammatoires, infectieuses et allergiques. Le matériau utilisé pour réaliser le stent sera prioritairement l'oxynitrure de titane. Le titane est par ailleurs mélangé à du nickel. Cet alliage possède plusieurs propriétés parmi les matériaux métalliques, entre autres la capacité de garder en mémoire une forme initiale et d'y retourner même après une déformation. C'est ce qu'on appelle plus communément les stents en alliage Nitinol^{®}. Cependant, vu la recherche constante dans le domaine des matériaux bio-compatibles, ainsi que l'essor progressif des nanotechnologies, tout autre matériel pourrait également, dans le futur, faire partie intégrante des stents. On pourrait également imaginer de travailler avec un stent partiellement siliconé, voire avec du matériel biodégradable, du cobalt-chrome, de l'or...

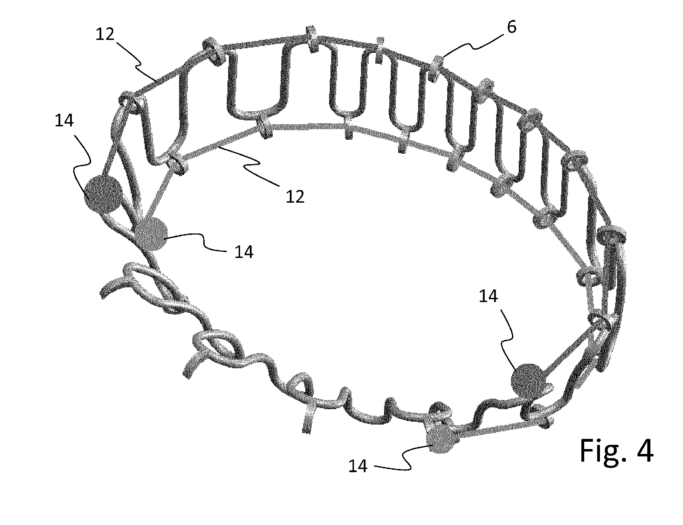

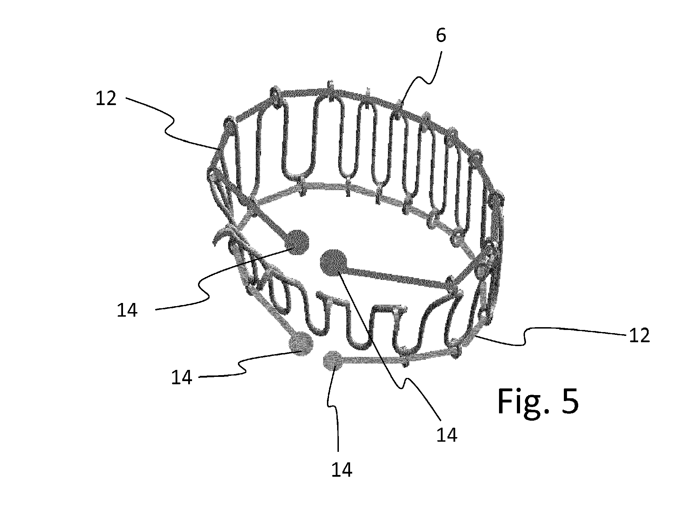

Les Fig. 4 et Fig. 5 montrent un mode de réalisation du stent muni d'une structure filaire pour rétracter le stent en vue de son retrait du pharynx du patient. Cette structure filaire comprend un fil 12 sur chacun des deux bords du stent. Chaque fil 12 traverse les anneaux 14 et est muni de deux perles 14 à ses extrémités, de manière à rester prisonnier des anneaux. Ces perles 14 sont également utilisées comme prises destinées à être sollicitées au retrait du stent. Le stent est en effet rétracté lorsque les billes 4 appartenant à un même fil sont rapprochées l'une de l'autre. La Fig. 4 correspond donc au stent lorsqu'il est dans son état déployé et la Fig. 5 représente le stent lorsque sa rétraction est sollicitée par un rapprochement mutuel des perles 4.

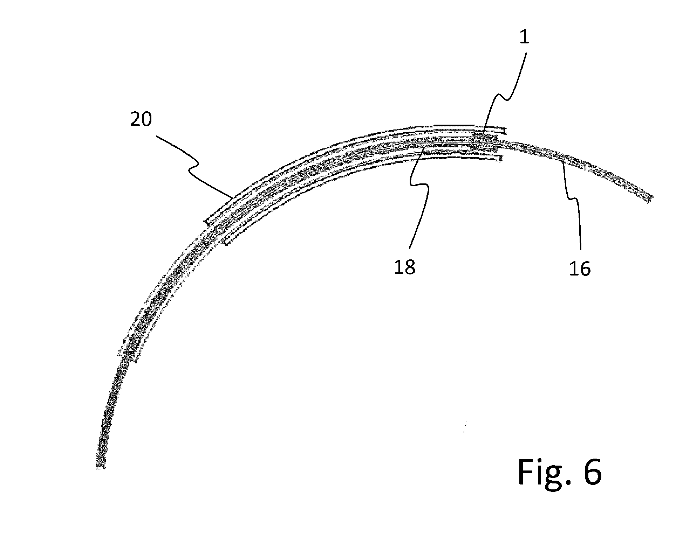

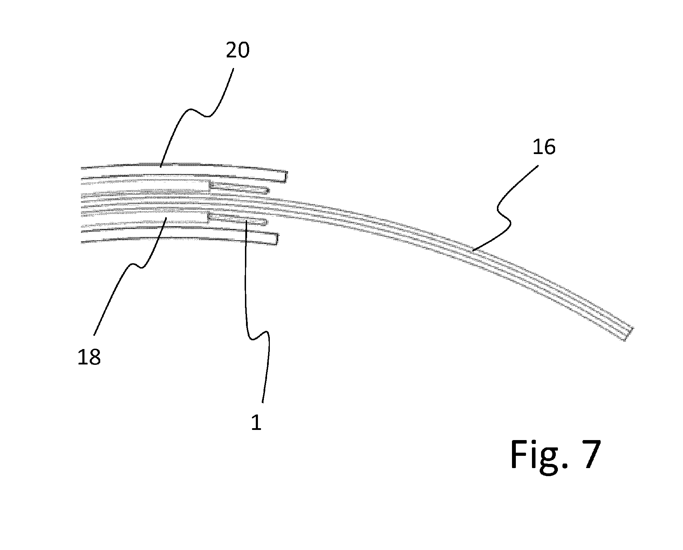

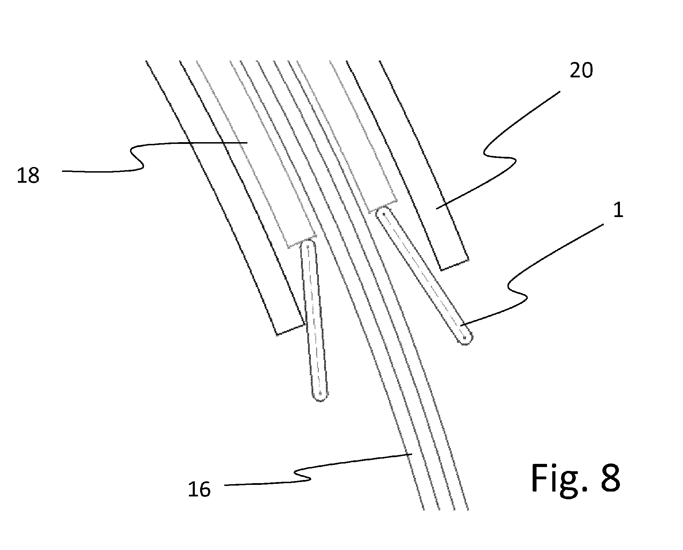

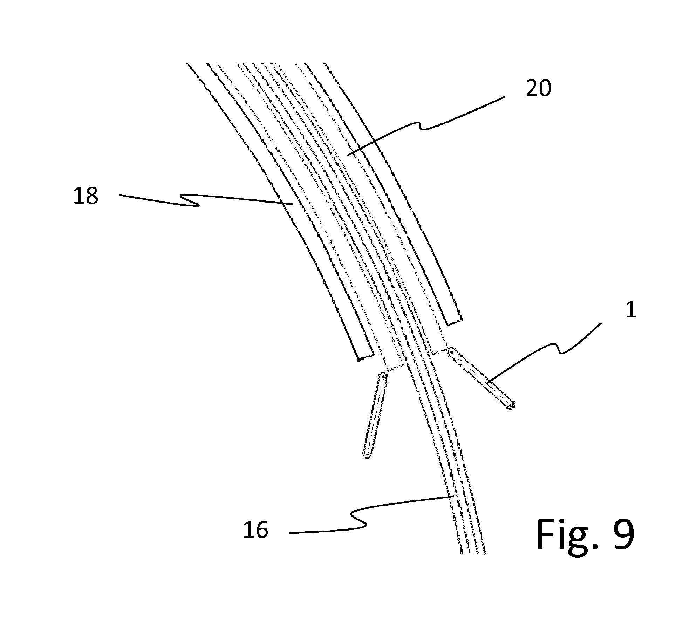

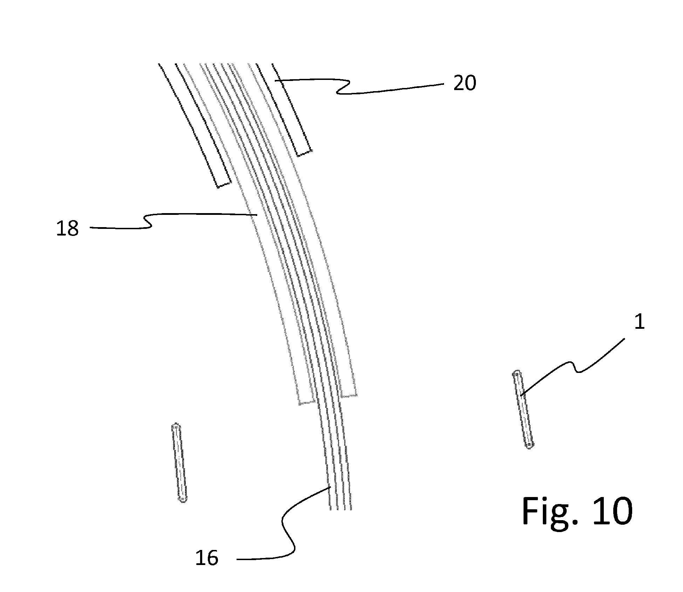

Les Fig. 6, 7, 8, 9 et 10 représentent le dispositif de largage selon l'invention. La Fig. 6 est une vue globale du dispositif de largage. Le stent pharyngé 1 et le tube poussoir 18 coulissent sur la sonde d'intubation 16. Une gaine externe 20 entoure étroitement le tube poussoir 18 et le stent 1. La sonde d'intubation 16 possède toutes les fonctionnalités d'une sonde d'intubation endotrachéale, de manière à alimenter le patient en oxygène durant l'anesthésie générale. Le stent pharyngé 1 et le tube poussoir 18 sont respectivement placés sur la partie distale et sur la partie proximale de la sonde d'intubation 16, de manière à pouvoir larguer le stent dans le pharynx du patient par un mouvement relatif entre le tube poussoir 18 et la gaine externe 20. Les Fig. 7, 8, 9 et 10 représentent plus précisément la partie distale du dispositif de largage selon l'invention à différents stades de largage du stent pharyngé. A la Fig. 7, le stent est encore bien en place sous la gaine externe 20. A la Fig. 8, suite à un mouvement relatif du tube poussoir 18 par rapport à la gaine externe 20 en direction de la partie distale de la gaine externe 20, le stent 1 est partiellement déployé. La Fig. 9 représente le moment où le stent est libéré de la gaine externe 20 pour être largué au sein de l'oropharynx. La Fig. 10 représente le dispositif de largage et le stent complètement déployé.

Il apparaîtra immédiatement pour l'homme du métier que la présente invention n'est pas limitée aux exemples illustrés et décrits ci-dessus. L'invention comprend chacune des caractéristiques nouvelles ainsi que leur combinaison. La présence de numéros de référence ne peut être considérée comme limitative. L'usage du terme « comprend » ne peut en aucune façon exclure la présence d'autres éléments autres que ceux mentionnés. L'usage de l'article défini « un » pour introduire un élément n'exclut pas la présence d'une pluralité de ces éléments. La présente invention a été décrite en relation avec des modes de réalisations spécifiques, qui ont une valeur purement illustrative et ne doivent pas être considérés comme limitatifs

## Revendications

1. Stent pharyngé pour traiter l'apnée du sommeil apte à exercer une contrainte prévenant l'effondrement des différents tissus en contact avec le stent **caractérisé en ce que** les propriétés d'adhésion varient sur la périphérie du stent, l'adhésion du stent étant adaptée aux propriétés des différents tissus en contact avec le stent.

2. Stent pharyngé selon la revendication 1 **caractérisé en ce que** l'adhésion du stent est adaptée par une variation de la contrainte locale exercée par le stent à sa périphérie sur les différents tissus, la contrainte locale exercée assurant une adhésion du stent aux différents tissus sans léser lesdits tissus.

3. Stent pharyngé selon la revendication 2, **caractérisé en ce que** la composante radiale de la contrainte locale exercée par le stent sur les tissus de la langue est supérieure à celle exercée sur les autres tissus du pharynx.

4. Stent pharyngé selon la revendication 3, **caractérisé en ce qu'il** comprend un maillage, les dimensions des mailles dudit maillage variant sur la périphérie du stent, le maillage de la partie du stent en contact avec la langue étant plus large que celui de la partie en contact avec les autres parois du pharynx.

5. Stent pharyngé selon la revendication 4, **caractérisé en ce que** le fil du maillage de la partie en contact avec la langue a une section d'aire plus élevée que le fil du maillage en contact avec les autres tissus du pharynx.

6. Stent pharyngé selon l'une des revendications 4 et 5, **caractérisé en ce que** des moyens additionnels pour assurer une bonne stabilité du stent sont prévus.

7. Stent pharyngé selon la revendication 6, **caractérisé en ce que** les moyens pour assurer une bonne stabilité du stent comprennent une incurvation (10) de la partie stent en contact avec la langue de manière à s'adapter à la morphologie de la langue.

8. Stent pharyngé selon l'une des revendications 6 et 7 **caractérisé en ce que** les moyens pour assurer une bonne stabilité du stent comprennent des crochets (8) fixés à la partie du stent en contact avec langue.

9. Stent pharyngé selon l'une des revendications précédentes **caractérisé en ce qu'il** comprend une structure filaire apte à étrangler le stent de manière à faciliter son retrait du pharynx.

10. Stent pharyngé selon la revendication 9 **caractérisé en ce que** la structure filaire comprend deux fils (12) traversant des anneaux (6) solidaires des mailles du stent, chacun des deux fils longeant un bord dudit stent pharyngé, les deux fils (12) étant munis de perles (14) en leurs extrémités.

11. Stent pharyngé (1) selon l'une des revendications précédentes **caractérisé en ce qu'il** est auto-expansible et est monté sur un dispositif de largage comprenant une sonde d'intubation (16), un tube poussoir (18), et une gaine tubulaire externe (20), ledit stent pharyngé (1) et le tube poussoir (18) étant aptes à coulisser sur la sonde d'intubation (16), le tube poussoir (18) se trouvant sur la partie proximale de la sonde d'intubation, le stent pharyngé (1) se trouvant sur la partie distale de la sonde d'intubation, la gaine externe (20) entourant ledit stent et ledit tube poussoir (18) et étant apte à coulisser sur ceux-ci, le diamètre dudit stent lorsqu'il est déployé étant supérieur au diamètre de la sonde d'intubation (16).
